# FASCICULE DE BREVET EUROPEEN

(11) **EP 4 566 574 B1**
(45) Date de publication et mention de la délivrance du brevet: **31.12.2025**
(21) Numéro de dépôt: 24218210.3
(22) Date de dépôt: 06.12.2024
(51) Int. Cl.: A61F 5/56

(54) **DISPOSITIF ORTHODONTIQUE**
ORTHODONTISCHE VORRICHTUNG
ORTHODONTIC DEVICE

(30) Priorité: 07.12.2023 FR 2313800
(43) Date de publication de la demande: 11.06.2025
(73) Titulaire: Orthogem Développement, 63000 Clermont-Ferrand (FR)
(72) Inventeur: MARIE-CATHERINE, Franck, 63000 CLERMONT-FERRAND (FR); MARIE-CATHERINE, Maxime, 63000 CLERMONT-FERRAND (FR)
(74) Mandataire: Ipsilon

(56) Documents cités:
- US-B1- 6 361 315
- US-B2- 8 267 093

## Description

### Domaine technique

La présente invention est dans le domaine de l'orthodontie. Elle concerne plus particulièrement un dispositif orthodontique apte à provoquer un déplacement d'une mandibule par rapport à un maxillaire et comportant notamment deux biellettes.

### Art antérieur

Différentes solutions ont été proposées pour provoquer un déplacement de la mandibule par rapport au maxillaire. Le but recherché est de déplacer la mandibule par rapport à sa position d'origine d'une très faible distance de l'ordre de quelques millimètres, dans un premier temps, afin, notamment, de stimuler la croissance. Cette propulsion mandibulaire peut être utilisée dans le cadre d'un traitement orthodontique de classe II ou de classe III, ou encore pour augmenter le flux respiratoire, afin de régler les problèmes de syndromes d'apnée du sommeil.

L'invention concerne plus particulièrement un ensemble orthodontique du type parfaitement connu pour un homme du métier, sous le nom de « orthèse de Herbst » ou « bielles de Herbst ».

Pour l'essentiel, ce type d'orthèse, illustré par exemple par le document US8267093, présente des gouttières coopérant, respectivement, avec la rangée de dents du haut et la rangée de dents du bas. Ces gouttières sont généralement réalisées dans un matériau rigide, par exemple en résine acrylique thermoformée, et sont reliées l'une à l'autre par un système de biellettes montées avec capacité de coulissement. Plus particulièrement, deux biellettes métalliques sont disposées, respectivement, de chaque côté de la gouttière supérieure et de la gouttière inférieure. Les deux biellettes sont reliées avec capacité d'articulation sur les gouttières habituellement par l'intermédiaire d'une armature filaire noyée dans la gouttière.

L'inconvénient qui en résulte, outre la nécessité d'utiliser du fil en chrome cobalt pour l'armature qui est une substance classée cancérogène, mutagène et reprotoxique (CMR), est la complexité de fabrication puisqu'il est nécessaire de fabriquer une armature métallique filaire et de la conformer en trois dimensions pour qu'elle s'adapte spécifiquement à la morphologie du patient. Une fois conformée, il est ensuite nécessaire de fabriquer les gouttières et d'y noyer ladite armature métallique pour assurer le maintien. L'une des difficultés réside dans le maintien de cette armature lors du coulage de la résine. Tout déplacement de cette armature, même minime, engendrerait une incompatibilité ou une gêne pour le patient, ou bien le dispositif n'assurerait pas le traitement optimal.

Une deuxième difficulté réside en ce qu'il est ensuite nécessaire de fixer le système d'articulation de la biellette à l'armature métallique filaire, notamment en réalisant des soudures / brasures de précision. Ce type de montage nécessite donc un niveau de compétences et de dextérité élevé pour le prothésiste dentaire qui doit ajuster de manière très précise en position et en orientation le système d'articulation sur l'armature filaire puis réaliser la soudre. Un décalage du système d'articulation lors de cette soudure pourrait également engendrer une incompatibilité ou une gêne pour le patient, ou un risque que le dispositif n'assure pas le traitement optimal.

### Exposé de l'invention

La présente invention vise à pallier les inconvénients de l'art antérieur en proposant un dispositif orthodontique parfaitement adapté à la morphologie du patient et à son programme de traitement, dont la fabrication est simple et rapide en permettant notamment d'éviter de réaliser des soudures / brasures de précision.

Un autre objectif est de diminuer, voire d'éviter l'utilisation de chrome cobalt.

Dans ce but, l'invention concerne un dispositif orthodontique apte à provoquer un déplacement d'une mandibule par rapport à un maxillaire, comportant une partie inférieure solidaire de ladite mandibule et une partie supérieure solidaire dudit maxillaire, reliées de manière articulée par deux biellettes télescopiques.

Selon l'invention, les parties supérieure et inférieure comprennent chacune une armature réalisée numériquement, c'est-à-dire de forme anatomique et en conception fabrication assistée par ordinateur (CFAO), pour entourer au moins partiellement une partie des dents du maxillaire et de la mandibule, chaque armature présentant une languette en saillie latéralement et vers l'extérieur de la maxillaire ou de la mandibule, la languette s'étendant dans un plan incliné d'un angle compris entre plus ou moins 10° par rapport à un plan d'occlusion entre la mandibule et le maxillaire, et de préférence le plan de la languette est parallèle au plan d'occlusion., La languette comprend un orifice comprenant des moyens d'accouplement avec un élément d'articulation de la biellette.

De cette manière, la fabrication du dispositif est simplifiée, et n'utilise pas d'armature filaire, ni ne met en œuvre des soudures de précision. L'armature réalisée numériquement s'adapte parfaitement à la morphologie du patient et permet de fixer les biellettes de manière précise et rapide. L'orientation des languettes et donc des éléments d'articulation qu'elles reçoivent est prédéterminée en fonction de la morphologie du patient de l'ampleur du déplacement mandibulaire à réaliser. L'élément d'articulation est reçu et fixé dans un orifice qui détermine sa position et son orientation. La fixation de l'élément d'articulation est donc simple, rapide et reproductible, et ne nécessite aucune compétence ou dextérité particulière, ni ne présente une quelconque difficulté.

En particulier, le fait que le plan de la languette soit parallèle au plan d'occlusion, avec une tolérance de plus ou moins 10° d'inclinaison dans n'importe quel sens permet des déplacements de la mandibule par rapport au maxillaire dans toutes les directions, pour traiter les déviations verticales (élévation ou abaissement), sagittales (protrusion ou rétrusion) de la mandibule par rapport au maxillaire, y compris les latéro-déviations afin d'éviter les troubles de l'articulation temporo-mandibulaire.

Dans cette configuration, plusieurs modes de réalisation peuvent être envisagés.

Les moyens d'accouplement peuvent être de tous types appropriés, par exemple par encliquetage, ou bien sont de préférence sous la forme d'un taraudage et l'élément d'articulation comprend un filetage.

Selon une forme de réalisation particulière, les moyens d'accouplement sont sous la forme d'un cylindre métallique fixé dans l'orifice de la languette qui, lui-même comprend des moyens d'accouplement avec l'élément d'articulation. Pour le montage, il convient alors simplement d'accoupler l'élément d'articulation avec le cylindre et de relier la biellette à l'élément d'articulation.

Afin de diminuer l'utilisation de métal et notamment de Chrome Cobalt, les armatures sont en résine, en polyétheréthercétone (PEEK), ou encore un polyaryléthercétone (PAEK) ou en plastique, et l'orifice de la languette reçoit le cylindre qui est maintenu dans l'orifice par de la résine ou de la colle, et lesdites armatures forment ainsi des gouttières.

La languette fait, de préférence, partie intégrante de l'armature et est donc réalisée dans la même matière.

Dans cette configuration, le cylindre comprend de préférence un méplat sur sa paroi latérale pour renforcer la solidarisation en rotation avec la colle ou la résine.

Selon une autre forme de réalisation, les armatures sont en métal, et l'orifice de la languette reçoit soit directement l'élément d'articulation, soit le cylindre maintenu dans l'orifice par au moins un point de soudure, et lesdites armatures sont recouvertes de résine pour former des gouttières. La fixation du cylindre n'implique qu'une soudure basique, sans aucun enjeu de précision de positionnement puisque le cylindre est maintenu dans un orifice qui détermine sa position et son orientation.

De préférence, le cylindre présente une tête épaulée pour venir prendre appui sur la languette.

Avantageusement, les biellettes sont articulées par rapport à l'élément d'articulation selon une amplitude d'environ 360° dans un plan horizontal et d'environ 90° au moins dans un plan vertical.

Selon une forme de réalisation particulière, l'élément d'articulation est par exemple une vis à tête sphérique autour de laquelle est montée et articulée une extrémité de la biellette.

### Brève description des dessins

[Fig. 1] est une vue illustrant les armatures pourvues de languettes mises en œuvre dans le dispositif selon l'invention, et montées respectivement sur un maxillaire et une mandibule.
[Fig. 2] est une vue similaire à celle de la figure 1, les armatures ayant été recouvertes de résine et des biellettes ayant été fixées aux languettes par l'élément d'articulation.
[Fig. 3] est une vue schématique en coupe d'une languette recevant un cylindre, lui-même recevant l'élément d'articulation de la biellette.
[Fig. 4] est une vue schématique en coupe d'un autre mode de réalisation illustrant une languette avec un orifice taraudé recevant directement l'élément d'articulation de la biellette.
[Fig. 5] est une vue du dispositif orthodontique de l'invention, mâchoires fermées.
[Fig. 6] est une vue du dispositif orthodontique de l'invention, mâchoires ouvertes.

### Description détaillée de l'invention

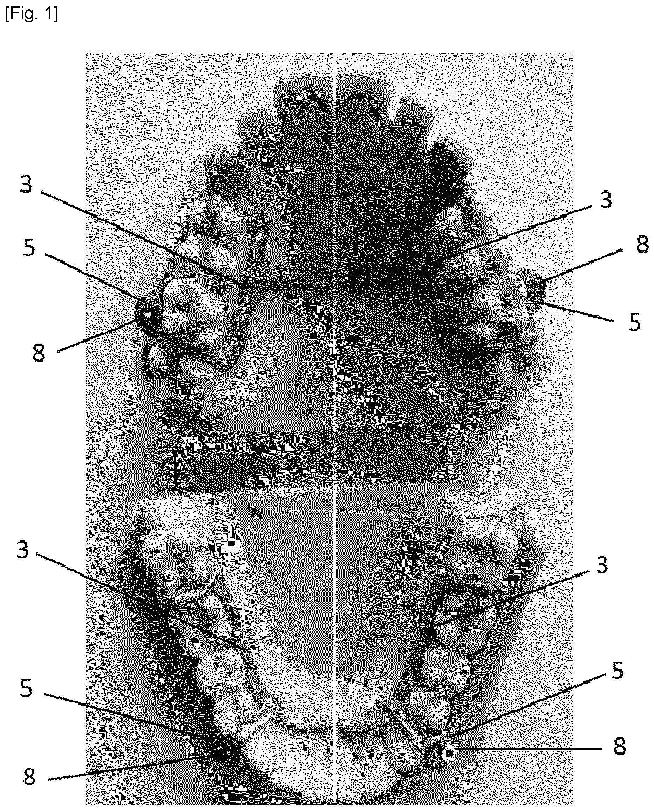

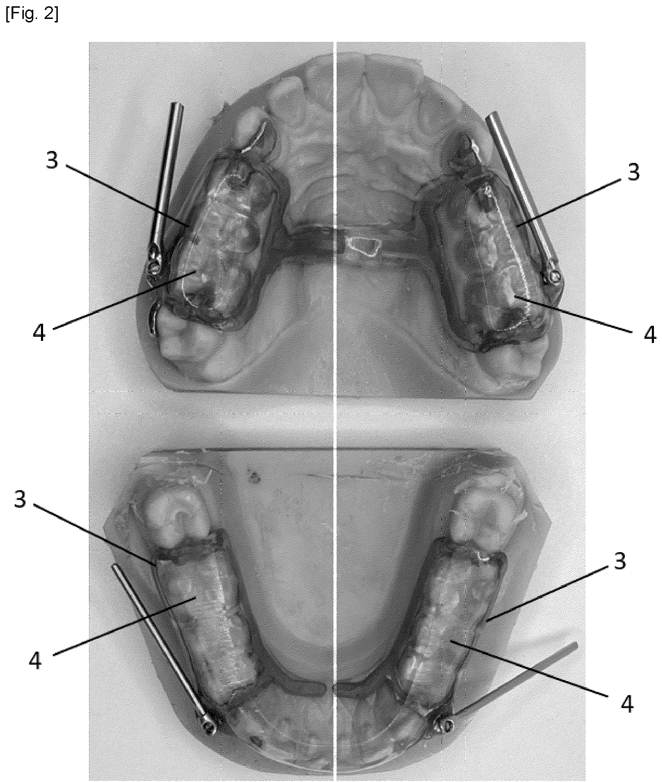

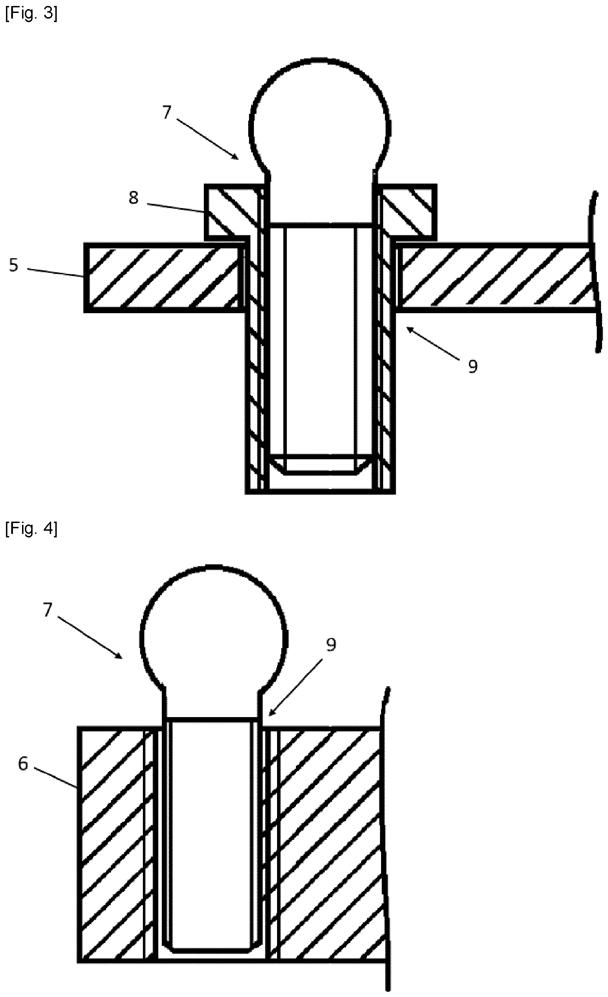

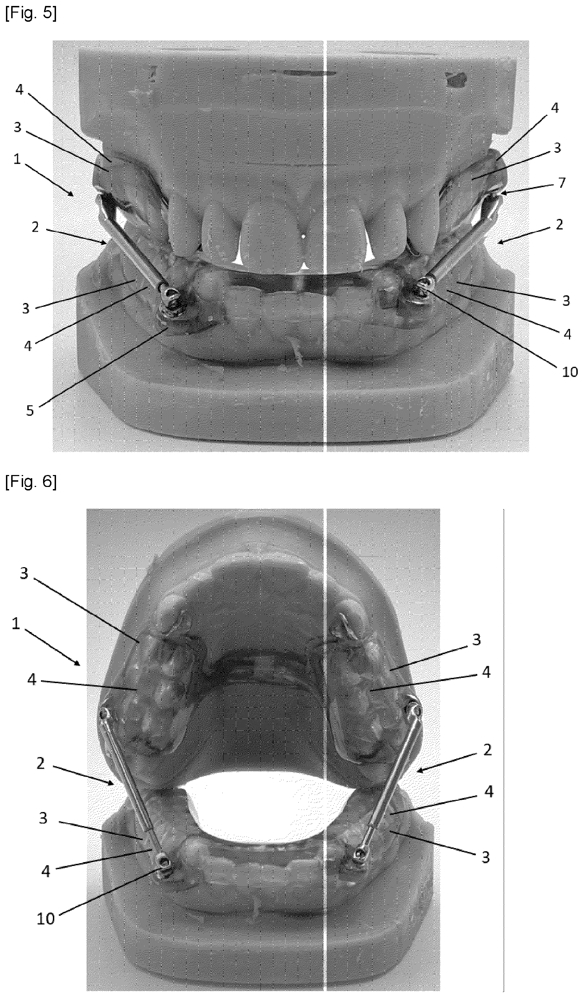

En référence aux figures 1 à 6, l'invention concerne un dispositif orthodontique (1), parfaitement adapté à la morphologie du patient, et apte à provoquer un déplacement d'une mandibule par rapport à un maxillaire, dont la fabrication est simple en permettant notamment d'éviter de réaliser des soudures / brasures de précision et d'éviter d'avoir recours à une armature filaire, et d'éviter de préférence l'utilisation de chrome cobalt.

Le dispositif orthodontique (1) comporte partie inférieure solidaire de ladite mandibule et une partie supérieure solidaire dudit maxillaire, reliées de manière articulée par deux biellettes (2) télescopiques.

Une première biellette (2) se trouve sur la droite du patient, par exemple sensiblement au niveau des premières molaires de droite pour le haut de la biellette (2), et par exemple sensiblement au niveau de la canine de droite pour le bas de la biellette (2). La seconde biellette (2) se trouve sur la gauche du patient, par exemple sensiblement au niveau des premières molaires de gauche pour le haut de la biellette (2), et par exemple sensiblement au niveau de la canine de gauche pour le bas de la biellette (2).

Dans le cadre de la présente invention, le terme « biellette » désigne un ensemble de deux éléments allongés, l'un coulissant dans l'autre afin que la longueur totale de la biellette (2) soit variable. Habituellement dans les dispositifs selon l'invention, c'est l'élément inférieur qui coulisse dans l'élément supérieur, sans que cela ne soit limitatif.

Dans le dispositif orthodontique (1) selon l'invention, les parties supérieure et inférieure comprennent chacune une armature (3) réalisée numériquement pour entourer au moins partiellement une partie des dents du maxillaire et de la mandibule.

Afin d'éviter l'utilisation de chrome cobalt, les armatures (3) peuvent être réalisées directement en résine, PEEK, PEAK ou plastique, dans ce cas elles recouvrent une plus grande partie des dents et forment directement des gouttières, ou bien être réalisées en métal et être ensuite recouvertes de résine (4) pour former les gouttières en tant que telles.

Selon l'invention, chaque armature (3) présent une languette (5) en saillie latéralement et vers l'extérieur du maxillaire ou de la mandibule. La languette (5) s'étend dans un plan incliné d'un angle compris entre plus ou moins 10° par rapport à un plan d'occlusion entre la mandibule et le maxillaire, et de préférence le plan de la languette est parallèle au plan d'occlusion. La languette (5) comprend un orifice (9) comprenant des moyens d'accouplement d'un élément d'articulation (7) de la biellette (2) de préférence selon une amplitude d'environ 360° dans un plan horizontal et d'environ 90° au moins dans un plan vertical. La languette (5) peut présenter une épaisseur comprise par exemple entre 0,5 et 5 mm.

Selon une forme de réalisation particulière, l'orifice (9) de la languette (5) reçoit un cylindre (8) métallique maintenu dans l'orifice (9) par de la résine ou de la colle lorsque l'armature (3) est réalisée en résine, PEEK, PEAK ou plastique, ou bien par au moins un point de soudure lorsque l'armature (3) est réalisée en métal, ou bien il peut être envisagé que l'orifice (9) de la languette (5) soit taraudé et que le cylindre (8) soit fileté. Le cylindre (8) présente par exemple une tête épaulée pour venir prendre appui sur la languette (5). Dans un mode de réalisation non illustré, l'orifice (9) de la languette (5) est étagé de sorte à recevoir la tête épaulée du cylindre (8) pour que le plan de la languette soit coplanaire avec celui de la tête épaulée. A titre d'exemple, la longueur du cylindre (8) est inférieure ou égale à l'épaisseur de la languette (5) pour éviter les risques de blessures du patient.

Dans le cas où le cylindre (8) est maintenu dans l'orifice (9) par de la colle ou de la résine, celui-ci comprend de préférence un méplat sur sa paroi latérale, non représenté sur les figures, pour éviter les mouvements de rotation.

Le cylindre (8) reçoit lui-même l'élément d'articulation (7) et comprend des moyens d'accouplement complémentaires à des moyens d'accouplement ménagés sur l'élément d'articulation (7). Ces moyens sont par exemple un taraudage réalisé dans le cylindre (8) et un filetage réalisé sur l'élément d'articulation (7).

Selon une autre forme de réalisation, et notamment lorsque les armatures (3) sont réalisées en métal, l'orifice (9) de la languette (5) peut recevoir directement l'élément d'articulation (7). Comme pour la version précédente, les moyens d'accouplement de l'orifice (9) sont par exemple un taraudage réalisé dans l'alésage (9) de la languette (5) et un filetage réalisé sur l'élément d'articulation (7).

Dans ces configurations, l'élément d'articulation (7) se présente par exemple sous la forme d'une vis à tête sphérique, formant une liaison rotule, autour de laquelle est montée et articulée une extrémité de la biellette (2).

En particulier, chaque extrémité d'une biellette (2) comprend une cage hémisphérique (10) avec un orifice débouchant destinée à recevoir en insertion la vis (7), de sorte que la cage (10) soit positionnée entre la languette (5) et la tête sphérique de la vis (7) pour former la liaison rotule afin de permettre une totale liberté d'articulation aux biellettes (2) tant dans un plan antéro-postérieur que dans un plan latéral, et ceci dans un encombrement réduit.

**Il** ressort de ce qui précède que l'invention fournit un dispositif orthodontique (1) parfaitement adapté à la morphologie du patient et à son programme de traitement, dont la fabrication est simple, rapide et reproductible en permettant notamment d'éviter de réaliser des soudures / brasures de précision et de diminuer, voire d'éviter d'avoir recours à du chrome cobalt.

## Revendications

1. Dispositif orthodontique (1) apte à provoquer un déplacement d'une mandibule par rapport à un maxillaire, comportant une partie inférieure solidaire de ladite mandibule et une partie supérieure solidaire dudit maxillaire, reliées de manière articulée par deux biellettes (2) télescopiques ***caractérisé* en ce que** les parties supérieure et inférieure comprennent chacune une armature (3) réalisée numériquement pour entourer au moins partiellement une partie des dents du maxillaire et de la mandibule, chaque armature (3) présentant une languette (5) en saillie latéralement et vers l'extérieur du maxillaire ou de la mandibule, la languette s'étend dans un plan incliné d'un angle compris entre plus ou moins 10° par rapport à un plan d'occlusion entre la mandibule et le maxillaire, et la languette (5) comprend un orifice (9) comprenant des moyens d'accouplement d'un élément d'articulation (7) de la biellette (2).

2. Dispositif orthodontique (1) selon la revendication 1, ***caractérisé* en ce que** la languette est parallèle au plan d'occlusion.

3. Dispositif orthodontique (1) selon l'une des revendications précédentes, ***caractérisé* en ce que** les moyens d'accouplement sont un taraudage et l'élément d'articulation (7) comprend un filetage.

4. Dispositif orthodontique (1) selon l'une des revendications précédentes, ***caractérisé* en ce que** les moyens d'accouplement sont sous la forme d'un cylindre (8) métallique fixé dans l'orifice (9) de la languette (5), le cylindre (8) comprend des moyens d'accouplement avec l'élément d'articulation (7).

5. Dispositif orthodontique (1) selon la revendication 4, ***caractérisé* en ce que** les armatures (3) sont en résine, PEEK, PEAK ou plastique, et l'orifice (9) de la languette (5) reçoit le cylindre (8) maintenu dans l'orifice (9) par de la résine ou de la colle, lesdites armatures (3) forment des gouttières.

6. Dispositif orthodontique (1) selon la revendication 5, ***caractérisé* en ce que** le cylindre (8) comprend un méplat sur sa paroi latérale.

7. Dispositif orthodontique (1) selon l'une des revendications 3 ou 4, ***caractérisé* en ce que** les armatures (3) sont en métal, et l'orifice (9) de la languette (5) reçoit soit directement l'élément d'articulation (7), soit le cylindre (8) maintenu dans l'orifice par au moins un point de soudure, lesdites armatures (3) sont recouvertes de résine (4) pour former des gouttières.

8. Dispositif orthodontique (1) selon la revendication 4, ***caractérisé* en ce que** le cylindre (8) présente une tête épaulée pour venir prendre appui sur la languette (5).

9. Dispositif orthodontique (1) selon l'une des revendications précédentes, ***caractérisé* en ce que** les biellettes (2) sont articulées par rapport à l'élément d'articulation (7) selon une amplitude d'environ 360° dans un plan horizontal et d'environ 90° au moins dans un plan vertical.

10. Dispositif orthodontique (1) selon la revendication 7, ***caractérisé* en ce que** l'élément d'articulation (7) est une vis à tête sphérique autour de laquelle est montée et articulée une extrémité de la biellette (2).

## Patentansprüche

1. Orthodontische Vorrichtung (1), die geeignet ist, eine Verlagerung eines Unterkiefers relativ zu einem Oberkiefer zu bewirken, umfassend einen unteren Teil, der mit dem Unterkiefer verbunden ist, und einen oberen Teil, der mit dem Oberkiefer verbunden ist, wobei die beiden Teile gelenkig durch zwei teleskopische Gestänge (2) miteinander verbunden sind, **dadurch gekennzeichnet, dass** der obere und der untere Teil jeweils einen digital hergestellten Rahmen (3) umfassen, der dazu ausgelegt ist, einen Teil der Zähne des Oberkiefers bzw. des Unterkiefers zumindest teilweise zu umschließen, wobei jeder Rahmen (3) eine seitlich und nach außen vom Oberkiefer oder Unterkiefer vorspringende Lasche (5) aufweist, die sich in einer Ebene erstreckt, die um einen Winkel von plus oder minus 10° relativ zu einer Okklusionsebene zwischen Unterkiefer und Oberkiefer geneigt ist, und wobei die Lasche (5) eine Öffnung (9) mit Kupplungsmitteln für ein Gelenkelement (7) des Gestänges (2) umfasst.

2. Orthodontische Vorrichtung (1) nach Anspruch 1, **dadurch gekennzeichnet, dass** die Lasche parallel zur Okklusionsebene verläuft.

3. Orthodontische Vorrichtung (1) nach einem der vorstehenden Ansprüche, **dadurch gekennzeichnet, dass** die Kupplungsmittel ein Gewinde umfassen und das Gelenkelement (7) ein entsprechendes Gegengewinde aufweist.

4. Orthodontische Vorrichtung (1) nach einem der vorstehenden Ansprüche, **dadurch gekennzeichnet, dass** die Kupplungsmittel in Form eines Metallzylinders (8) ausgebildet sind, der in der Öffnung (9) der Lasche (5) befestigt ist, wobei der Zylinder (8) Kupplungsmittel mit dem Gelenkelement (7) aufweist.

5. Orthodontische Vorrichtung (1) nach Anspruch 4, **dadurch gekennzeichnet, dass** die Rahmen (3) aus Harz, PEEK, PAEK oder Kunststoff bestehen und die Öffnung (9) der Lasche (5) den Zylinder (8) aufnimmt, der mittels Harz oder Klebstoff in der Öffnung (9) gehalten wird, wobei die Rahmen (3) Aligner-/Schienen bilden.

6. Orthodontische Vorrichtung (1) nach Anspruch 5, **dadurch gekennzeichnet, dass** der Zylinder (8) an seiner Seitenwand eine Abflachung aufweist.

7. Orthodontische Vorrichtung (1) nach einem der Ansprüche 3 oder 4, **dadurch gekennzeichnet, dass** die Rahmen (3) aus Metall bestehen und die Öffnung (9) der Lasche (5) entweder direkt das Gelenkelement (7) oder den Zylinder (8) aufnimmt, wobei der Zylinder (8) durch mindestens einen Schweißpunkt in der Öffnung gehalten wird, und die Rahmen (3) mit Harz (4) überzogen sind, um Schienen zu bilden.

8. Orthodontische Vorrichtung (1) nach Anspruch 4, **dadurch gekennzeichnet, dass** der Zylinder (8) einen Schulterkopf aufweist, der sich gegen die Lasche (5) abstützt.

9. Orthodontische Vorrichtung (1) nach einem der vorstehenden Ansprüche, **dadurch gekennzeichnet, dass** die Gestänge (2) relativ zu dem Gelenkelement (7) mit einem Bewegungsumfang von etwa 360° in einer Horizontalebene und von mindestens etwa 90° in einer Vertikalebene gelenkig ausgebildet sind.

10. Orthodontische Vorrichtung (1) nach Anspruch 7, **dadurch gekennzeichnet, dass** das Gelenkelement (7) eine Schraube mit kugelförmigem Kopf ist, um den ein Ende des Gestänges (2) montiert und gelenkig gelagert ist.

## Claims

1. Orthodontic device (1) configured to cause displacement of a mandible relative to a maxilla, comprising a lower part secured to said mandible and an upper part secured to said maxilla, the two parts being articulately connected by two telescopic connecting rods (2),
**characterized in that** the upper and lower parts each comprise a digitally manufactured framework (3) configured to at least partially surround a portion of the teeth of the maxilla and mandible, each framework (3) having a tab (5) protruding laterally outward from the maxilla or mandible, the tab extending in a plane inclined by an angle of plus or minus 10° relative to an occlusal plane between the mandible and the maxilla, and the tab (5) comprising an orifice (9) including coupling means for an articulation element (7) of the connecting rod (2).

2. Orthodontic device (1) according to claim 1, **characterized in that** the tab is parallel to the occlusal plane.

3. Orthodontic device (1) according to any one of the preceding claims, **characterized in that** the coupling means are a threading, and the articulation element (7) comprises a complementary thread.

4. Orthodontic device (1) according to any one of the preceding claims, **characterized in that** the coupling means are in the form of a metal cylinder (8) fixed in the orifice (9) of the tab (5), the cylinder (8) comprising coupling means with the articulation element (7).

5. Orthodontic device (1) according to claim 4, **characterized in that** the frameworks (3) are made of resin, PEEK, PAEK, or plastic, and the orifice (9) of the tab (5) receives the cylinder (8) held in the orifice (9) by resin or adhesive, the frameworks (3) thereby forming trays.

6. Orthodontic device (1) according to claim 5, **characterized in that** the cylinder (8) comprises a flat on its lateral wall.

7. Orthodontic device (1) according to any one of claims 3 or 4, **characterized in that** the frameworks (3) are made of metal, and the orifice (9) of the tab (5) receives either the articulation element (7) directly or the cylinder (8), the cylinder being held in the orifice by at least one weld point, the frameworks (3) being coated with resin (4) to form trays.

8. Orthodontic device (1) according to claim 4, **characterized in that** the cylinder (8) has a shouldered head designed to bear against the tab (5).

9. Orthodontic device (1) according to any one of the preceding claims, **characterized in that** the connecting rods (2) are articulated relative to the articulation element (7) with a range of motion of approximately 360° in a horizontal plane and at least approximately 90° in a vertical plane.

10. Orthodontic device (1) according to claim 7, **characterized in that** the articulation element (7) is a screw with a spherical head around which one end of the connecting rod (2) is mounted and articulated.
